# FASCICULE DE BREVET EUROPEEN

(11) **EP 3 157 599 B1**
(45) Date de publication et mention de la délivrance du brevet: **11.09.2019**
(21) Numéro de dépôt: 15736559.4
(22) Date de dépôt: 22.06.2015
(51) Int. Cl.: A61M 11/00, A61B 5/00, A61K 49/00

(54) **PROCÉDÉ DE RÉALISATION, DANS UNE SALLE D'EXPOSITION, D'UNE ATMOSPHÈRE CHARGÉE EN ALLERGÈNES, HOMOGÈNE ET À PARAMÈTRES CHOISIS**
VERFAHREN ZUR ERZEUGUNG EINER HOMOGENEN ALLERGENBELASTETEN ATMOSPHÄRE MIT AUSGEWÄHLTEN PARAMETERN IN EINEM AUSSETZUNGSRAUM
METHOD FOR CREATING A HOMOGENEOUS ALLERGEN-LADEN ATMOSPHERE WITH CHOSEN PARAMETERS IN AN EXPOSURE ROOM

(30) Priorité: 23.06.2014 FR 1455821
(43) Date de publication de la demande: 26.04.2017
(73) Titulaire: Alyatec, 67000 Strasbourg (FR); Hôpitaux Universitaires de Strasbourg (HUS), 67000 Strasbourg (FR)
(72) Inventeur: DE BLAY, Frédéric, F-67100 Strasbourg (FR); SANTAILLER, Gérard, F-69480 Marcy (FR)
(74) Mandataire: Merckling, Norbert
(86) Numéro de dépôt international: PCT/FR2015/051649
(87) Numéro de publication internationale: WO 2015/197962

(56) Documents cités:
- EP-B1- 1 335 750
- WO-A1-2007/140601
- WO-A1-2010/063714
- JP-A- 2010 063 967
- US-A- 5 030 253
- US-A1- 2009 145 466
- US-A1- 2009 155 146
- US-A1- 2009 257 919
- W. EDUARD ET AL: "Generation and Homogeneity of Aerosols in a Human Whole-Body Inhalation Chamber", ANNALS OF OCCUPATIONAL HYGIENE, vol. 52, no. 6, 7 juillet 2008 (2008-07-07), pages 545-554, XP055134071, ISSN: 0003-4878, DOI: 10.1093/annhyg/men039
- C. LIDÈN ET AL: "A New Whole-body Exposure Chamber for Human Skin and Lung Challenge Experiments-the Generation of Wheat Flour Aerosols", ANNALS OF OCCUPATIONAL HYGIENE, vol. 42, no. 8, 1 novembre 1998 (1998-11-01), pages 541-547, XP055134038, ISSN: 0003-4878, DOI: 10.1093/annhyg/42.8.541
- CHRISTIAN MONSÉ ET AL: "Considerations for the design and technical setup of a human whole-body exposure chamber", INHALATION TOXICOLOGY, vol. 24, no. 2, 1 janvier 2012 (2012-01-01), pages 99-108, XP055134058, ISSN: 0895-8378, DOI: 10.3109/08958378.2011.640362
- J. H. DAY ET AL: "The role of allergen challenge chambers in the evaluation of anti-allergic medication: an international consensus paper", CLINICAL <HTML_ENT GLYPH="@AMP;" ASCII="&"/> EXPERIMENTAL ALLERGY REVIEWS, vol. 6, no. 2, 1 février 2006 (2006-02-01) , pages 31-59, XP055133981, ISSN: 1472-9725, DOI: 10.1111/j.1365-2222.2005.00099.x

## Description

La présente invention concerne un procédé de réalisation d'une atmosphère chargée en allergènes homogène, dont la teneur en allergènes et la taille de ces allergènes sont choisies et contrôlées, dans une salle d'exposition aux allergènes destinée à accueillir des patients en vue d'une provocation allergique chez ces patients.

La salle d'exposition aux allergènes est une enceinte confinée, de type dit EEC (appelée Environmental Exposure Chamber ou European Exposure Chamber), connue également sous le nom de chambre de test d'allergie.

Les allergies sont un fléau mondial qui concerne plus d'une personne sur quatre en Occident. On estime que d'ici quelques années, près de 50 % de la population des pays développés pourrait être touchée par au moins une maladie allergique. La recherche médicale dans le domaine de l'allergie et notamment celle se rapportant au développement de médicaments antiallergiques ou de traitements de désensibilisation, est de ce fait un secteur qui se développe considérablement.

Pour réaliser des études cliniques relatives à l'allergie ou pour évaluer l'efficacité de nouveaux médicaments ou de traitement de désensibilisation, il est indispensable d'observer la réaction de patients allergiques lorsqu'ils se retrouvent exposés aux allergènes naturels.

La réalisation de telles observations en milieu naturel, de façon scientifique et objective, est très difficile en raison des importantes fluctuations de la quantité d'allergènes inhalés par un patient en fonction, par exemple, de la saison, des conditions climatiques ou des endroits fréquentés par le patient. En effet, la quantité d'allergènes naturellement présents dans l'air ambiant est très variable selon par exemple la région où l'on se trouve, la saison, les conditions météorologiques notamment la température, l'humidité ou la présence de vent, le moment de la journée, ou même la hauteur par rapport au niveau du sol.

Pour pouvoir s'affranchir de ces très nombreux paramètres qui fluctuent de façon difficilement contrôlable, on a développé dans l'art antérieur des dispositifs appelés chambres de test d'allergie ou EEC (European/Environmental Exposure Chamber).

Ces dispositifs permettent d'accueillir un ou plusieurs patients dans un lieu clos où l'on diffuse une quantité contrôlée d'allergènes et d'observer scientifiquement leur réaction physiologique après un temps d'exposition plus ou moins long. Les différents paramètres expérimentaux au sein de la chambre doivent être constants et contrôlés tout au long de l'expérimentation. On peut ainsi réaliser des tests dans des conditions reproductibles sur plusieurs patients ou plusieurs fois sur un même patient et obtenir immédiatement et de façon fiable des résultats comparables les uns avec les autres.

De tels dispositifs rendent possibles des tests d'inhalation d'allergènes sous conditions contrôlées se voulant proches de l'état naturel et reproductibles. Ils visent à garantir des résultats fiables, complets, comparables et utilisables scientifiquement dans le cadre d'études cliniques. Ils sont ainsi utilisables par exemple pour tester in vivo l'efficacité de traitements de désensibilisation ou de nouveaux médicaments antiallergiques et par exemple pour déterminer les doses à prescrire ou la durée d'efficacité.

On connaît ainsi par exemple la chambre de test d'allergie décrite dans le brevet européen n° EP 1335750 au nom de HORAK ou celles décrites dans les demandes de brevet FRAUNHOFER WO 2010/063714 et PATEL WO 2007/140601.

Dans ces dispositifs antérieurs connus, une préoccupation constante concerne l'obtention d'une concentration en allergènes homogène dans toute la salle d'exposition. En effet, pour obtenir des résultats fiables et exploitables, il est très important de s'assurer que les patients sont exposés à la même concentration d'allergènes quelle que soit leur position dans la salle. Différents dispositifs de distribution et de répartition des allergènes dans la salle d'exposition ont donc été imaginés à cet effet dans l'art antérieur.

Dans le brevet HORAK, on introduit d'une part la poudre d'allergène au moyen d'un distributeur de particules solides à air comprimé placé dans le plafond de la salle d'exposition et d'autre part, de l'air frais dépourvu d'allergènes au moyen de buses sphériques, distinctes du distributeur de particules, également situées au plafond mais à des endroits différents et orientées dans des directions différentes afin de provoquer un courant d'air turbulent.

Les turbulences résultantes de l'air frais introduit entraînent les particules solides d'allergènes en suspension et provoquent la distribution de ces dernières dans la salle d'exposition.

Un tel système de distribution n'est cependant pas très satisfaisant. En effet, il est très difficile d'homogénéiser des particules introduites sous forme sèche. Les particules d'allergènes solides sont entraînées par leur poids et ont tendance à retomber vers le sol, créant ainsi un gradient de concentration en allergènes en fonction de la hauteur par rapport au niveau du sol.

Pour pallier ces inconvénients, on a décidé dans les brevets FRAUNHOFER et PATEL d'utiliser une préparation liquide d'allergènes à partir de laquelle on génère un aérosol. Une fois introduit dans la salle d'exposition, cet aérosol est mélangé, au moyen d'un ou plusieurs ventilateurs, à l'air ambiant se trouvant dans la salle d'exposition et provenant d'entrées d'air distinctes.

Là encore, ces dispositifs présentent de graves inconvénients. L'utilisation de ventilateurs est particulièrement désavantageuse. En effet, la rotation des pales des ventilateurs génère des ondes de chocs mécaniques susceptibles de dégrader les allergènes qui sont des composés particulièrement fragiles.

En outre, un ventilateur crée un brassage de l'air erratique et non maîtrisable. La répartition de l'air brassé n'est pas homogène dans l'espace. On trouve sous le ventilateur une colonne neutre dans laquelle le brassage est quasi inexistant, celui-ci étant au contraire maximal en périphérie du ventilateur. Une concentration homogène dans le temps et l'espace n'est donc absolument pas garantie avec de tels dispositifs.

On connait également la chambre d'exposition décrite dans la demande de brevet JP 2010 063967 qui vise également à fournir un dispositif capable de maintenir une concentration uniforme et constante d'une substance à l'intérieur d'une salle d'exposition. Pour cela, la substance est envoyée dans la salle d'exposition à travers une multitude d'ouvertures situées dans le plafond de celle-ci, puis est aspirée hors de la salle d'exposition à travers une multitude d'ouvertures situées dans le plancher de celle-ci. La substance aspirée retourne vers le dispositif d'injection pour être de nouveau envoyée dans la salle d'exposition, le dispositif fonctionnant en circuit fermé.

Avec un tel recyclage de la substance injectée, il est impossible de garantir une concentration constante dans le temps car la quantité de substance présente dans les rejets n'est pas maîtrisable.

En outre, pour être injectée, la substance passe à travers les pales d'un ventilateur ce qui risque de fortement endommager une substance fragile telle que des allergènes. Tous ces dispositifs antérieurs ne permettent pas de garantir l'obtention dans toute la salle d'exposition d'une concentration en allergènes prédéfinie, contrôlée, homogène et sensiblement constante. Elles ne permettent donc pas de régler la quantité d'allergènes inhalée par un patient pendant son exposition et de s'assurer que tous les patients présents dans la salle d'exposition inhalent la même quantité d'allergènes quelle que soit leur position dans la salle d'exposition.

Or l'obtention de cet objectif est une condition indispensable pour pouvoir garantir la reproductibilité des résultats mesurés et pouvoir valablement comparer les effets de l'exposition aux allergènes sur les différents patients ou d'une séance à l'autre. La fiabilité et la validité scientifique des études cliniques menées au moyen des chambres de test d'allergie de l'art antérieur peuvent donc être mises en doute.

En outre, aucuns de ces dispositifs de l'art antérieur ne s'intéresse à la taille des particules d'allergènes envoyées dans la salle d'exposition. Or, il s'agit d'un critère très important en ce qui concerne le déclenchement des réactions physiologiques allergiques chez les patients.

Il existe donc un besoin réel pour un dispositif et un procédé qui permettent d'atteindre de tels objectifs dans une chambre de test d'allergie de ce type.

C'est précisément ce que fournit l'invention en enseignant un dispositif et un procédé qui permettent de générer, dans une salle d'exposition une atmosphère chargée en allergènes dont la teneur et la taille des particules sont homogènes dans toute la salle, stables pendant toute la durée de l'exposition, contrôlées et correspondent aux valeurs préalablement choisies.

Le procédé selon l'invention permet avantageusement, et de façon complètement inédite dans l'art antérieur, de maîtriser et de contrôler l'exposition aux allergènes à laquelle seront soumis les patients présents dans la salle d'exposition, tant au niveau de la diffusion des allergènes, c'est-à-dire de la concentration en allergènes de l'air brassé dans la salle d'exposition, qu'au niveau du profil de ces allergènes, c'est-à-dire de la répartition en taille des particules d'allergènes diffusées exprimée en MMAD (« Mass Médian Aerodynamic diameter » en anglais ou « diamètre aérodynamique médian en masse » en français).

Selon l'étude réalisée et l'allergène étudié, des paramètres d'exposition sont préalablement fixés en ce qui concerne la diffusion et le profil des allergènes à obtenir dans la salle d'exposition ceci afin de refléter par exemple les conditions d'exposition réelles à cet allergène dans la nature.

Le procédé selon l'invention permet avantageusement de garantir que ces paramètres préalablement choisis seront obtenus avec des tolérances raisonnables dans toute la salle d'exposition et pendant toute la durée de l'exposition.

Pour cela l'invention fournit un procédé de réalisation, dans une salle d'exposition aux allergènes, d'une atmosphère chargée en allergènes, homogène et dont la concentration en allergènes Cₑₓₚₒₛᵢₜᵢₒₙ et le MMAD (diamètre aérodynamique médian en masse) des allergènes Dₑₓₚₒₛᵢₜᵢₒₙ sont préalablement choisis et contrôlés.

Selon l'invention, le procédé est mis en oeuvre au moyen d'un dispositif de nébulisation par ultrasons, à partir d'une composition aqueuse d'allergènes et d'un flux d'air dépourvu d'allergènes présentant un débit dₐᵢᵣ constant.

Le procédé selon l'invention comprend les étapes suivantes :
- on choisit les valeurs Cₑₓₚₒₛᵢₜᵢₒₙ et Dₑₓₚₒₛᵢₜᵢₒₙ que l'on souhaite obtenir à une tolérance près dans l'atmosphère de la salle d'exposition,
- en fonction de la valeur Dₑₓₚₒₛᵢₜᵢₒₙ choisie, on détermine la fréquence F à laquelle on doit faire fonctionner le dispositif de nébulisation par ultrasons et la concentration en allergènes C_{composition} que doit présenter la composition aqueuse d'allergènes, pour obtenir dans l'atmosphère de la salle d'exposition un MMAD des allergènes correspondant à la valeur Dₑₓₚₒₛᵢₜᵢₒₙ choisie à la tolérance près,
- en fonction de la valeur Cₑₓₚₒₛᵢₜᵢₒₙ choisie, du débit dₐᵢᵣ que présente le flux d'air dépourvu d'allergènes, du rendement du dispositif de nébulisation par ultrasons et de la concentration en allergènes C_{composition} déterminée, on détermine le débit d_{composition} avec lequel on doit introduire la composition aqueuse d'allergènes dans le dispositif de nébulisation par ultrasons, pour obtenir dans l'atmosphère de la salle d'exposition une concentration en allergènes correspondant à la valeur Cₑₓₚₒₛᵢₜᵢₒₙ choisie à la tolérance près,
- on fournit ou on prépare la composition aqueuse d'allergènes avec la concentration en allergènes C_{composition} déterminée,
- on introduit avec le débit d_{composition} déterminé la composition aqueuse d'allergènes dans le dispositif de nébulisation par ultrasons fonctionnant à la fréquence F déterminée,
- on produit, à partir de la composition aqueuse d'allergènes et au moyen du dispositif de nébulisation par ultrasons, un nébulisat humide, formé de gouttelettes de composition aqueuse d'allergènes en suspension dans l'air,
- on sèche au moyen d'un flux d'air de séchage le nébulisat humide afin d'obtenir un nébulisat sec, formé de particules d'allergènes solides en suspension dans l'air,
- on mélange, en dehors de la salle d'exposition, le nébulisat sec au flux d'air dépourvu d'allergènes présentant le débit dₐᵢᵣ, pour obtenir un flux d'air chargé en allergènes,
- on introduit dans la salle d'exposition le flux d'air chargé en allergènes, à travers au moins une bouche de diffusion située en partie haute de la salle d'exposition,
- on aspire le flux d'air chargé en allergènes hors de la salle d'exposition à travers au moins une bouche d'évacuation située en partie basse de la salle d'exposition.

En outre, selon un mode de réalisation préférentiel de l'invention, on réalise des mesures particulaires en continu afin de surveiller le non-dépassement de seuils de sécurité.

Par ailleurs, selon un mode de réalisation préférentiel de l'invention, on réalise une procédure de qualification qui comprend au moins une des étapes suivantes :
- on mesure la concentration et le MMAD des allergènes dans l'atmosphère de la salle d'exposition afin de contrôler qu'ils correspondent respectivement aux valeurs Cₑₓₚₒₛᵢₜᵢₒₙ et Dₑₓₚₒₛᵢₜᵢₒₙ choisies à la tolérance près ;
- on mesure le rendement du dispositif de nébulisation par ultrasons dans les conditions de fonctionnement correspondant à la mise en oeuvre du procédé ;
- on utilise plusieurs bouches d'évacuation réparties à différents endroits en partie basse de la salle d'exposition et on choisit le nombre, l'emplacement et/ou l'ouverture des bouches d'évacuation utilisées pour aspirer le flux d'air chargé en allergènes, afin d'obtenir une diffusion homogène dudit flux d'air chargé en allergènes dans l'ensemble de la salle d'exposition.

Selon un mode de réalisation préférentiel du procédé, on prépare la composition aqueuse d'allergènes avec la concentration en allergènes C_{composition} déterminée, et on vérifie sa concentration en allergènes par la technique ELISA.

Selon un mode de réalisation préférentiel du procédé, la mesure du rendement du dispositif de nébulisation par ultrasons se fait par prélèvement du nébulisat sec pendant une durée déterminée ; par mise en solution du nébulisat sec prélevé puis mesure de la concentration C_{prélèvement} de cette solution par la technique ELISA ; par calcul de la quantité d'allergènes se trouvant dans le prélèvement à partir de la concentration C_{prélèvement} mesurée ; et par comparaison de la quantité d'allergènes se trouvant dans le prélèvement avec la quantité d'allergènes introduite dans le dispositif de nébulisation par ultrasons pendant la même durée, calculée à partir de la concentration C_{composition} et du débit d_{composition}.

Selon un mode de réalisation préférentiel du procédé, la mesure de la concentration des allergènes dans l'atmosphère de la salle d'exposition est réalisée par prélèvement aéraulique puis par dosage du prélèvement par la technique ELISA.

Selon un mode de réalisation préférentiel du procédé, on procède à des ajustements de la concentration en allergènes C_{composition} ou du débit d_{composition}, si la concentration ou le MMAD des allergènes mesurés dans l'atmosphère de la salle d'exposition pendant la procédure de qualification ne correspondent pas respectivement aux valeurs Cₑₓₚₒₛᵢₜᵢₒₙ ou Dₑₓₚₒₛᵢₜᵢₒₙ choisies à la tolérance près.

Selon un mode de réalisation préférentiel du procédé, on mesure au niveau de chaque fauteuil équipant la salle d'exposition, la concentration des allergènes dans l'atmosphère de la salle d'exposition ou la dose totale de particules d'allergènes reçues à cet endroit pendant la durée de l'exposition.

L'invention enseigne également un système d'exposition aux allergènes comprenant une salle d'exposition aux allergènes, et apte à produire dans cette salle d'exposition une atmosphère chargée en allergènes, homogène et dont la concentration en allergènes Cₑₓₚₒₛᵢₜᵢₒₙ et le MMAD (diamètre aérodynamique médian en masse) des allergènes Dₑₓₚₒₛᵢₜᵢₒₙ peuvent être préalablement choisis.

Ce système d'exposition aux allergènes comporte :
- un dispositif de nébulisation par ultrasons, réglé pour travailler à une fréquence F adaptée par rapport à la valeur de Dₑₓₚₒₛᵢₜᵢₒₙ choisie, et comprenant une zone de préparation d'un nébulisat humide à partir d'une composition aqueuse d'allergènes, et une entrée d'air de séchage par laquelle un flux d'air de séchage est amené au contact du nébulisat humide pour le sécher et former ainsi un nébulisat sec d'allergènes;
- une composition aqueuse d'allergènes préparée ou fournie avec une concentration C_{composition} dépendant de la valeur Dₑₓₚₒₛᵢₜᵢₒₙ choisie et de la fréquence F;
- un moyen d'introduction, avec un débit réglable, de la composition aqueuse d'allergènes dans le dispositif de nébulisation par ultrasons ;
- une zone de mélange, séparée de la salle d'exposition, communiquant d'une part avec la sortie du dispositif de nébulisation par ultrasons et d'autre part avec une arrivée d'un flux d'air dépourvu d'allergènes de débit constant, et dans laquelle est réalisé le mélange entre le nébulisat sec et le flux d'air dépourvu d'allergènes pour obtenir un flux d'air chargé en allergènes;
- au moins une bouche de diffusion située en partie haute de la salle d'exposition, qui communique avec la chambre de mélange et par laquelle le flux d'air chargé en allergènes pénètre dans la salle d'exposition ;
- plusieurs bouches d'évacuation, permettant d'aspirer le flux d'air chargé en allergènes hors de la salle d'exposition, réparties à différents endroits en partie basse de la salle d'exposition, et dont l'ouverture peut être fermée et réglée indépendamment les unes des autres.

Selon un mode de réalisation de l'invention, le système d'exposition aux allergènes comporte au moins un dispositif de mesure particulaire permettant de surveiller en continu le non-dépassement de seuils de sécurité ou de mesurer le MMAD des allergènes dans l'atmosphère de la salle d'exposition.

Ce dispositif de mesure particulaire est préférentiellement un compteur de particules par laser ou par caméra.

Selon un mode de réalisation de l'invention, le système d'exposition aux allergènes comporte un collecteur d'aérosol qui permet de réaliser un prélèvement du nébulisat sec par prélèvement aéraulique, ou une plaque froide qui permet de réaliser un prélèvement du nébulisat sec par condensation de celui-ci.

Selon un mode de réalisation de l'invention, le système d'exposition aux allergènes comporte au moins un dispositif de mesure de la concentration des allergènes dans l'atmosphère de la salle d'exposition.

D'autres caractéristiques et avantages de l'invention apparaitront à la lecture de la description détaillée qui va suivre, description faite en référence aux dessins annexés, dans lesquels :
- la figure 1 est une vue de dessus schématique d'ensemble d'un exemple de système d'exposition aux allergènes selon l'invention ;
- la figure 2 est une vue schématique en coupe transversale d'un exemple de système d'exposition aux selon l'invention ;
- la figure 3 est un organigramme général illustrant un exemple de procédé selon l'invention.

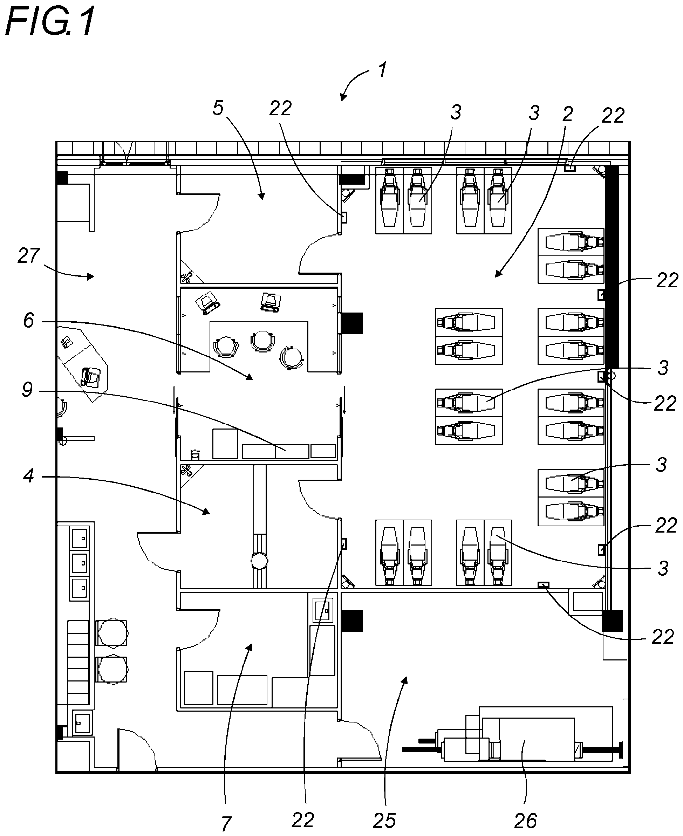

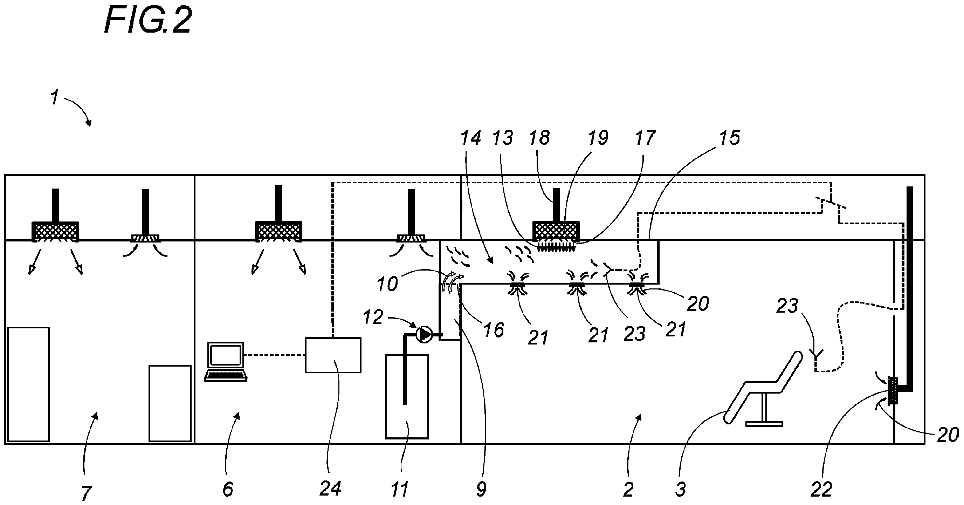

On a représenté sur les figures 1 et 2 le plan général d'un exemple de réalisation d'un système d'exposition aux allergènes 1 selon l'invention.

Ce système d'exposition aux allergènes 1 comprend plusieurs pièces, dont une salle d'exposition 2 qui est destinée à accueillir les patients à observer et dans laquelle on peut réaliser, en présence ou l'absence de patients, par le procédé selon l'invention une atmosphère à teneur contrôlée en allergènes et dont la répartition en taille est parfaitement maîtrisée.

La salle d'exposition 2 est une enceinte confinée, dont la pression ambiante est inférieure à la pression de référence (qui est celle des locaux environnant le système d'exposition 1) et à celle du reste du système d'exposition 1.

La taille et la forme de la salle d'exposition 2 dépendent du lieu d'implantation du système, des contraintes techniques à respecter et des souhaits des exploitants.

Elle renferme un ou plusieurs fauteuils 3 dans lesquels les patients peuvent s'installer confortablement pour toute la durée de l'exposition.

Un sas d'entrée 4 et un sas de sortie 5, en surpression par rapport à la salle d'exposition 2 et par rapport à l'extérieur, permettent d'entrer et de sortir de la salle d'exposition 2 sans qu'il y ait de contamination par les allergènes à l'extérieur du système d'exposition aux allergènes 1. Ces sas 4 et 5 permettent également d'empêcher les polluants extérieurs (autres allergènes, composés chimiques) d'entrer dans la salle d'exposition 2.

Une salle de contrôle 6, également en surpression par rapport à la salle d'exposition 2 et par rapport à l'extérieur, permet aux opérateurs de venir régler et contrôler les différents paramètres d'expérimentation et de surveiller la salle d'exposition 2.

Le système d'exposition aux allergènes 1 peut comporter en outre un laboratoire 7 dans lequel on prépare une composition aqueuse d'allergènes 8 utilisée dans le cadre du procédé selon l'invention. Ce laboratoire 7 est équipé de manière à permettre cette préparation en toute sécurité.

Le système 1 comporte également un dispositif de nébulisation par ultrasons 9, qui est par exemple disposé dans la salle de contrôle 6 et qui permet de préparer un nébulisat sec 10 d'allergènes à partir de la composition aqueuse d'allergènes 8 initialement sous forme liquide.

Selon un mode de réalisation préférentiel de l'invention, le dispositif de nébulisation par ultrasons 9 est un atomiseur par ondes capillaires.

Il comprend une zone de préparation 34 d'un nébulisat humide à partir de la composition aqueuse d'allergènes 8, et une entrée d'air de séchage 35 par laquelle un flux d'air de séchage 30 est amené au contact du nébulisat humide pour le sécher et former ainsi le nébulisat sec 10 d'allergènes. Sur l'exemple représenté sur la figure 2, la composition aqueuse d'allergènes 8 préparée dans le laboratoire 7 est versée dans un réservoir 11, puis envoyée dans le dispositif de nébulisation par ultrasons au moyen d'une pompe 12.

Le nébulisat sec 10 obtenu est mélangé à un flux d'air dépourvu d'allergènes 13 en dehors de la salle d'exposition, dans une zone de mélange séparée de la salle d'exposition 2 et formée de préférence d'une chambre de mélange 14.

Il s'agit d'un volume creux, délimité par des parois 15 qui forment un caisson, distinct et séparé de la salle d'exposition 2 et également distinct et séparé du conduit d'arrivée d'air, par exemple situé dans le faux-plafond partiellement au-dessus de la salle d'exposition 2 et de la salle de contrôle 6.

Cette chambre de mélange 14 comporte au moins une entrée d'allergènes 16, qui communique avec la sortie du dispositif de nébulisation par ultrasons 9 et par laquelle pénètre le nébulisat sec 10, et au moins une entrée d'air 17 par laquelle arrive l'air dépourvu d'allergènes 13 destiné à venir ventiler la salle d'exposition 2.

Cette entrée d'air 17 communique avec un conduit d'arrivée d'air 18 préférentiellement équipé d'un dispositif de filtration 19 adapté, par exemple un caisson filtrant encastrable ou en gaine, qui empêche que d'autres allergènes, particules indésirables ou même certains polluants chimiques ne pénètrent dans la chambre de mélange 14 et par la suite dans la salle d'exposition 2.

La taille de ce dispositif de filtration 19, notamment sa surface filtrante, est définie en fonction du débit dₐᵢᵣ du flux d'air dépourvu d'allergènes 13 entrant et donc du volume de la salle d'exposition 2 à ventiler.

En effet, le débit dₐᵢᵣ du flux d'air dépourvu d'allergènes 13 doit être proportionnel au volume de la salle d'exposition 2, afin de garantir un taux de brassage satisfaisant dans la salle d'exposition, et préférentiellement conforme à la norme 14644-1 permettant ainsi d'obtenir pour la salle d'exposition 2 un classement particulaire hors exposition de classe ISO8.

Dans la chambre de mélange 14, le flux d'air 13 qui arrive par l'entrée d'air 17 débouche dans une zone d'entrée dont la section est plus importante que celle du conduit d'arrivée d'air 18. Elle constitue ainsi un élargissement par rapport à ce conduit d'arrivée d'air 18, qui provoque une détente par expansion du flux d'air dépourvu d'allergènes 13 lorsqu'il pénètre dans la chambre. La vitesse du flux d'air 13 est donc ralentie.

Le flux d'air 13 ralenti vient ensuite entraîner le nébulisat sec 10 qui arrive par l'entrée d'allergènes 16.

Du fait de la disposition relative des entrées d'air 17 et d'allergènes 16, de la forme et de la taille limitée de la chambre de mélange 14, de la mise en pression de la chambre de mélange 14 par l'entrée de l'air dépourvu d'allergènes 13 et de la réduction de vitesse du flux d'air 13 à son entrée dans la chambre de mélange, il se crée au sein de la chambre de mélange 14 des micro-turbulences qui permettent de réaliser un mélange doux et satisfaisant du nébulisat sec 10 avec le flux d'air dépourvu d'allergènes 13, sans qu'il soit nécessaire d'ajouter un dispositif de brassage tel qu'un ventilateur ou autre.

Par ce mélange du nébulisat sec 10 et du flux d'air dépourvu d'allergènes 13, on obtient un flux d'air chargé en allergènes 20 qui est introduit dans la salle d'exposition 2 à travers une ou plusieurs bouches de diffusion 21 qui se trouvent en partie haute de la salle d'exposition.

Le nombre, la taille, la disposition et l'orientation de ces bouches de diffusion 21 sont choisis par l'homme du métier en fonction de la taille, de la capacité d'accueil et de la forme de la salle d'exposition 2. En fonction des paramètres géométriques de la salle d'exposition 2, la ou les bouches de diffusion 21 sont disposées de manière à assurer une distribution homogène du flux d'air chargés en particules d'allergènes 20 dans toute la salle d'exposition 2, sans direction préférentielle, ni zone privilégiée.

Ces bouches de diffusion 21 peuvent éventuellement être équipées d'une grille ou de tout autre dispositif approprié permettant d'orienter ou de régler le flux d'air chargé en particules d'allergènes 20 sans le casser, ni endommager les particules d'allergènes.

Selon l'invention, la salle d'exposition 2 est équipée de plusieurs bouches d'évacuation 22 qui permettent d'aspirer le flux d'air chargé en allergènes 20 hors de la salle d'exposition 2 de manière à mettre en circulation l'air présent dans la salle d'exposition 2 et ainsi à réaliser une ventilation de cette salle d'exposition 2.

Ces bouches d'évacuation 22 sont disposées à différents endroits en partie basse de la salle d'exposition 2 et sont préférentiellement réparties tout autour de la salle d'exposition 2.

Ces bouches d'évacuation 22 présentent une ouverture réglable indépendamment les unes des autres, par exemple au moyen d'une vanne à iris. Elles peuvent ainsi être, chacune et indépendamment les unes des autres, complètement fermées, complètement ouvertes ou partiellement ouvertes avec un degré d'ouverture choisi, afin d'homogénéiser la distribution du flux dans toute la salle d'exposition 2.

Le système d'exposition aux allergènes 1 représenté comporte également des capteurs tels que 23, disposés aux endroits appropriés du système et par exemple dans la salle d'exposition 2, préférentiellement au niveau de chaque fauteuil 3, et/ou dans la chambre de mélange 14 selon la chaîne métrologique qui sera détaillée ci-après. Ces capteurs 23 sont reliés à des dispositifs de mesure tels que 24 par exemple situés dans la salle de contrôle 6.

Il peut ainsi s'agir par exemple d'un capteur de température, de pression ou d'humidité, d'un capteur relié à un dispositif de mesure particulaire ou d'un dispositif de prélèvement, tel que par exemple un collecteur d'aérosol ou une plaque froide de condensation, ou de tout autre capteur utile pour surveiller, contrôler ou piloter le déroulement du procédé selon l'invention.

Il peut s'agir également d'un dispositif 36 de mesure de la concentration des allergènes dans l'atmosphère de la salle d'exposition, par exemple sous la forme d'un dispositif de mesure portatif permettant de réaliser la mesure à un endroit quelconque de la salle d'exposition, ou encore d'un ou plusieurs dispositifs de mesure fixe situés à proximité ou au niveau d'un, de plusieurs ou de chaque fauteuil équipant la salle d'exposition.

Comme représenté sur la figure 1, le système d'exposition aux allergènes 1 peut comprendre en outre un local technique 25 dans lequel se trouvent par exemple les appareils 26 nécessaires à la ventilation, l'humidification, la climatisation et/ou le chauffage des différentes pièces du système d'exposition aux allergènes 1 ou tout autre appareillage ou matériel encombrant nécessaire au fonctionnement du système 1.

L'ensemble pourra être complété par une salle d'accueil et d'attente 27 pour les patients.

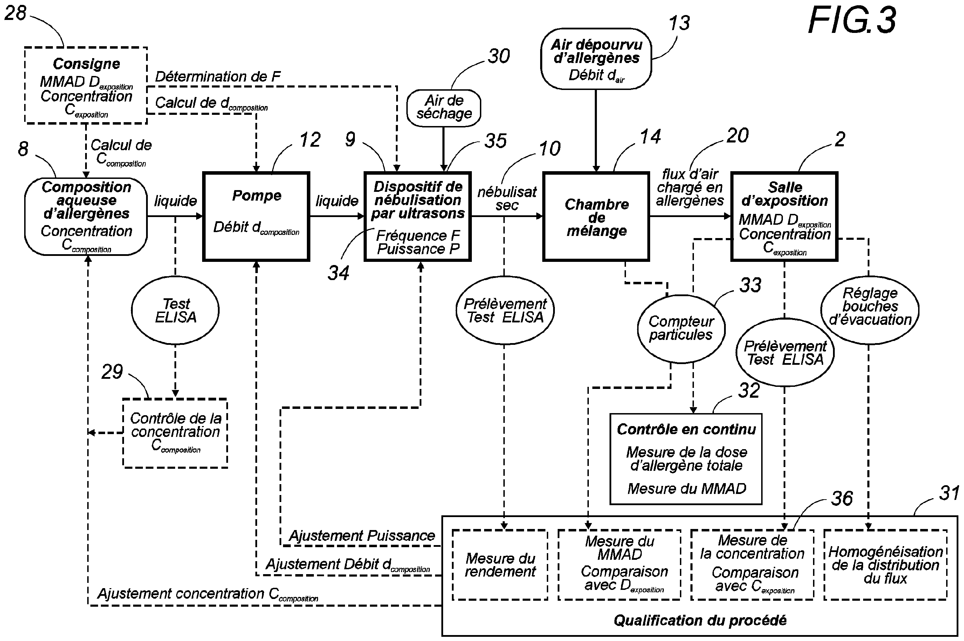

Ce système d'exposition aux allergènes 1 permet de mettre en oeuvre le procédé selon l'invention que l'on va décrire plus en détails ci-dessous en référence à l'organigramme de la figure 3.

Lorsque l'on veut réaliser dans la salle d'exposition 2 une atmosphère chargée en allergènes, homogène et dont les paramètres relatifs aux allergènes sont choisis et contrôlés, on peut avantageusement utiliser le procédé selon l'invention qui nécessite, comme mentionné précédemment, l'utilisation d'un dispositif de nébulisation par ultrasons 9, d'une composition aqueuse d'allergènes 8 et d'un flux d'air dépourvu d'allergènes 13 de débit dₐᵢᵣ imposé par le volume de la salle d'exposition 2.

Pour cela, on commence par définir une consigne 28 concernant les paramètres relatifs aux allergènes que l'on souhaite obtenir dans l'atmosphère qui sera réalisée dans la salle d'exposition 2. On choisit ainsi la concentration en allergènes, appelée Cₑₓₚₒₛᵢₜᵢₒₙ, et le diamètre aérodynamique médian en masse ou MMAD des allergènes, appelé Dₑₓₚₒₛᵢₜᵢₒₙ, que l'on souhaite obtenir dans la salle d'exposition 2 à la tolérance près.

On peut ainsi choisir par exemple pour Cₑₓₚₒₛᵢₜᵢₒₙ une valeur sensiblement égale à 60 ng/m³ d'air diffusé avec une tolérance de 20%, et pour Dₑₓₚₒₛᵢₜᵢₒₙ une valeur sensiblement égale à 10 µm avec une tolérance de 20%.

En fonction des valeurs de consigne choisies, on détermine ensuite les différents paramètres de fonctionnement du système qui permettront d'atteindre cette consigne.

A partir de la valeur Dₑₓₚₒₛᵢₜᵢₒₙ choisie, on détermine la fréquence F à laquelle on doit faire fonctionner le dispositif de nébulisation par ultrasons 9 et la concentration en allergènes C_{composition} que doit présenter la composition aqueuse d'allergènes 8.

Il a en effet été démontré que lorsque l'on nébulise de l'eau au moyen d'un dispositif de nébulisation par ultrasons la taille des gouttelettes d'eau obtenues dépend uniquement de la fréquence de travail du dispositif de nébulisation.

Lorsque l'on remplace l'eau par une composition aqueuse de particules en solution ou en suspension dans l'eau, les particules sont entraînées dans ces gouttelettes d'eau nébulisées, formant ainsi un nébulisat humide. Après séchage de ce nébulisat humide, on obtient un nébulisat sec 10 qui ne contient plus que les particules en suspension dans l'air.

Dans ce nébulisat sec, la taille des particules en suspension dépend de la taille des gouttelettes d'eau du nébulisat humide (donc de la fréquence de travail du dispositif de nébulisation) et de la concentration de ces particules dans la composition aqueuse initiale.

Dans le cadre du procédé selon l'invention, la valeur Dₑₓₚₒₛᵢₜᵢₒₙ choisie pour le MMAD permet de déterminer la gamme de fréquence à prévoir pour le dispositif de nébulisation 9 et de choisir un dispositif fonctionnant à une fréquence F adaptée par rapport à cette gamme.

A partir des valeurs Dₑₓₚₒₛᵢₜᵢₒₙ choisie et F déterminée, on peut calculer la concentration C_{composition} que doit présenter la composition aqueuse d'allergènes 8 avant son introduction dans le dispositif de nébulisation 9.

Pour obtenir dans la salle d'exposition 2 une concentration en allergènes correspondant à la valeur Cₑₓₚₒₛᵢₜᵢₒₙ choisie à la tolérance près, il suffit alors de calculer le débit d_{composition} avec lequel on doit introduire la composition aqueuse d'allergènes 8 dans le dispositif de nébulisation par ultrasons 9, à l'aide de la valeur Cₑₓₚₒₛᵢₜᵢₒₙ choisie, du débit dₐᵢᵣ que présente le flux d'air dépourvu d'allergènes 13, du rendement du dispositif de nébulisation par ultrasons 9 (qui peut être estimé, calculé, déduit de données techniques communiquées par le fabricant, ou préférentiellement déterminé dans une procédure préalable de qualification du procédé comme on le verra par la suite) et de la concentration en allergènes C_{composition} calculée précédemment.

Une fois les paramètres de fonctionnement déterminés, on fournit ou on prépare la composition aqueuse d'allergènes 8 avec une concentration qui correspond à la valeur C_{composition} calculée.

Selon la nature de l'allergène utilisé, cette composition peut être une solution ou une suspension de particules d'allergènes dans de l'eau ou éventuellement dans du sérum physiologique.

Elle est préparée préférentiellement à partir d'un extrait d'allergènes lyophilisé, ou d'un extrait liquide d'allergènes.

Une fois sa préparation réalisée avec la concentration en allergènes C_{composition} déterminée, ou lorsqu'une composition aqueuse d'allergène est directement fournie à la bonne concentration, on peut avantageusement vérifier sa concentration en allergènes par la technique ELISA. Un ajustement de concentration peut ainsi être réalisé si nécessaire avant de passer à l'étape suivante du procédé. Cette étape de contrôle de la concentration est référencée 29 sur l'organigramme de la figure 3.

On introduit ensuite la composition aqueuse d'allergène 8 de concentration C_{composition} dans le dispositif de nébulisation par ultrasons 9 avec le débit d_{composition} calculé. On utilise pour cela tout moyen approprié permettant d'introduire la composition avec un débit choisi et préférentiellement réglable. On utilisera par exemple une pompe 12 à débit réglable, notamment une pompe péristaltique et de préférence une pompe péristaltique avec un moteur contrôlé pas à pas qui permet de contrôler avec précision le débit du liquide introduit.

Le dispositif de nébulisation par ultrasons 9, qui est de préférence un atomiseur par ondes capillaires, est réglé pour travailler à la fréquence F préalablement déterminée avec une puissance P dépendant du matériel utilisé.

A l'aide de ce dispositif de nébulisation par ultrasons 9, on produit à partir de la composition aqueuse d'allergènes 8 introduite, un nébulisat humide formé de gouttelettes de composition aqueuse d'allergènes en suspension dans l'air.

Un flux d'air de séchage 30, de faible débit, est envoyé sur ce nébulisat humide afin de l'entraîner et de le sécher par évaporation de l'eau contenue dans les gouttelettes. Après séchage, on obtient un nébulisat sec 10, formé de particules d'allergènes solides en suspension dans l'air.

On mélange ce nébulisat sec 10 au flux d'air dépourvu d'allergènes 13 prévu pour ventiler la salle d'exposition 2 avec le débit dₐᵢᵣ. On obtient ainsi un flux d'air chargé en allergènes 20.

Ce mélange est réalisé en dehors de la salle d'exposition 2 et de préférence dans une chambre de mélange 14 séparée de la salle d'exposition 2.

Le débit du flux d'air de séchage 30 étant faible, le débit du nébulisat sec 10 est négligeable par rapport à celui du flux d'air dépourvu d'allergènes 13. Le flux d'air chargé en allergènes 20 obtenu présente donc également un débit sensiblement égal à dₐᵢᵣ.

On introduit ce flux d'air chargé en allergènes 20 dans la salle d'exposition 2, à travers la ou les bouches de diffusion 21 qui sont situées en partie haute de la salle d'exposition 2, et on l'aspire à nouveau hors de la salle d'exposition 2 à travers les bouches d'évacuation 22 qui se trouvent réparties en partie basse de la salle d'exposition 2, assurant ainsi la ventilation de la salle d'exposition 2.

Grâce au procédé selon l'invention, le flux d'air chargé en allergènes 20 qui est introduit dans la salle d'exposition 2 présente les caractéristiques initialement choisies comme consigne, c'est-à-dire une concentration en allergènes correspondant à Cₑₓₚₒₛᵢₜᵢₒₙ à la tolérance près et un diamètre aérodynamique médian en masse (MMAD) des allergènes correspondant à Dₑₓₚₒₛᵢₜᵢₒₙ à la tolérance près.

L'obtention de ces valeurs de consigne dans la salle d'exposition 2 est préférentiellement contrôlée par une chaîne métrologique complète, pouvant comporter à la fois des mesures effectuées lors d'une procédure préalable de qualification du procédé 31 et des mesures de contrôle en continu 32.

Préalablement, on réalise préférentiellement une procédure de qualification qui permet de calibrer le procédé et le système 1 selon l'invention et d'ajuster les différents paramètres de fonctionnement de ce système en fonction des résultats obtenus lors de cette procédure de qualification 31.

Pendant cette procédure de qualification, on peut notamment améliorer l'homogénéisation de la distribution du flux d'air chargé en allergènes 20 dans la salle d'exposition 2 par un réglage de l'ouverture des bouches d'évacuation 22.

On règle ainsi indépendamment chacune des bouches d'évacuation 22 tout en maintenant constant le débit total d'air aspiré par l'ensemble des bouches d'évacuation 22 afin qu'il reste conforme à la valeur totale d'aspiration prédéfinie pour garantir un taux de brassage satisfaisant de la salle d'exposition 2 et une mise en dépression de cette salle par rapport au reste du système 1 pour des raisons de sécurité.

On peut choisir les bouches d'évacuation 22 qui seront utilisées et celles qui seront fermées, ainsi que le degré d'ouverture de chacune des bouches d'évacuation 22 afin d'obtenir dans la salle d'exposition 2 une diffusion la plus homogène possible sans direction, ni zone privilégiée. L'homogénéisation de la diffusion est ainsi optimisée en fonction des particularités géométriques spécifiques de la salle d'exposition 2.

Lors de la procédure de qualification, on peut également mesurer le rendement du dispositif de nébulisation par ultrasons 9 dans les conditions de fonctionnement qui correspondront à l'exposition particulière envisagée, c'est-à-dire avec la puissance et la fréquence déterminées pour le dispositif de nébulisation 9 et avec la nature de l'allergène, la concentration C_{composition} et le débit d_{composition} qui seront utilisés pour cette exposition spécifique.

Pour mesurer ce rendement, on peut préférentiellement réaliser un prélèvement du nébulisat sec 10 pendant une durée déterminée, mettre en solution le nébulisat sec 10 prélevé puis mesurer la concentration C_{prélèvement} de cette solution par la technique ELISA. On calcule ensuite la quantité d'allergènes se trouvant dans le prélèvement à partir de la concentration C_{prélèvement} mesurée et on compare la quantité d'allergènes se trouvant dans le prélèvement avec la quantité d'allergènes introduite dans le dispositif de nébulisation par ultrasons 9 pendant la même durée, calculée à partir de la concentration C_{composition} et du débit d_{composition}.

Le prélèvement du nébulisat sec 10 s'effectue après le dispositif de nébulisation par ultrasons 9, tout de suite à sa sortie ou dans la chambre de mélange 14, lorsque le système d'exposition aux allergènes 1 en comporte une, de préférence à l'entrée de celle-ci.

Le prélèvement du nébulisat sec 10 peut être réalisé par tout moyen approprié imaginable par l'homme du métier et par exemple par prélèvement aéraulique au moyen d'un collecteur d'aérosol ou par condensation du nébulisat sec 10 sur une plaque froide. Le prélèvement obtenu est ensuite dosé en laboratoire par la technique ELISA.

Dans le premier cas, le nébulisat sec 10 est aspiré pendant une durée précise déterminée puis conduit sur un filtre adapté pour retenir les particules d'allergènes. Ce filtre est ensuite élué et la solution résultante est dosée par la méthode ELISA.

Dans le second cas, on introduit une plaque froide que l'on interpose sur le trajet du nébulisat sec pendant une durée précise déterminée, puis on retire cette plaque froide et on récupère la solution condensée sur la plaque afin de la soumettre à un dosage chimique de type ELISA.

En comparant la quantité d'allergènes présente dans le prélèvement déduite des résultats de l'analyse ELISA à la quantité d'allergènes introduite dans le dispositif de nébulisation par ultrasons 9 pendant la même durée précise, on peut calculer le rendement du dispositif de nébulisation par ultrasons 9 dans ces conditions particulières de fonctionnement..

Pendant la procédure de qualification, on contrôle que l'on arrive à obtenir, avec les paramètres de fonctionnement déterminés, les valeurs fixées par consigne dans la salle d'exposition.

On mesure ainsi la concentration en allergènes dans la salle d'exposition 2 afin de contrôler qu'elle correspond à la valeur Cₑₓₚₒₛᵢₜᵢₒₙ choisie à la tolérance près.

Pour cela, on réalise un prélèvement dans la salle d'exposition par tout moyen approprié imaginable par l'homme du métier et de préférence par prélèvement aéraulique au moyen d'un collecteur d'aérosol comme expliqué précédemment. Le prélèvement obtenu est ensuite dosé en laboratoire par la technique ELISA.

Cette mesure peut être réalisée à un endroit quelconque de la salle d'exposition 2, par exemple au moyen d'un dispositif de mesure portatif.

La mesure dans la salle d'exposition 2 de la concentration en allergènes de l'atmosphère d'inhalation peut, en outre ou alternativement, être réalisée au niveau de chaque fauteuil 3 afin d'avoir une mesure locale de cette concentration et de pouvoir réaliser une cartographie générale de l'exposition.

On réalise également des mesures particulaires par tout moyen approprié et préférentiellement au moyen d'un ou plusieurs compteurs de particules 33 adaptés à la taille des allergènes. Il s'agit préférentiellement d'un compteur optique de particules, préférentiellement par laser et par exemple un compteur de particules six canaux ou un spectromètre de mesure du diamètre aérodynamique des particules, ou encore d'un compteur de particules par caméra.

Ces compteurs de particules permettent de mesurer le taux particulaire (nombre de particules par m³) et la distribution granulométrique (taille des particules). Un traitement statistique de ces résultats permet d'aboutir au MMAD des particules.

Les mesures sont réalisées à l'aide d'une ou plusieurs sondes de captage, préférentiellement des sondes isocinétiques, disposées aux points de mesure souhaités et reliées par un tuyau à un compteur de particules 33. Sur l'exemple de réalisation représenté sur la figure 2, il s'agit par exemple des capteurs 23 reliés au dispositif de mesure 24.

Ces mesures particulaires sont effectuées à un ou plusieurs endroits du système d'exposition aux allergènes 1, de préférence dans la chambre de mélange 14 et dans la salle d'exposition 2, préférentiellement au niveau de chaque fauteuil 3.

Ces mesures particulaires sont réalisées pendant la procédure de qualification 31 afin de calibrer le système en vérifiant que le MMAD des allergènes présents dans l'atmosphère de la salle d'exposition correspond bien à la valeur de consigne Dₑₓₚₒₛᵢₜᵢₒₙ choisie à la tolérance près.

Elles peuvent avantageusement être également réalisées en continu pendant le fonctionnement du système d'exposition aux allergènes 1 en présence ou en absence de patients, afin de surveiller en continu qu'on ne dépasse pas certains seuils de sécurité. En cas de dépassement, des procédures de sécurité peuvent être déclenchées si nécessaire, comme par exemple l'arrêt du procédé ou l'évacuation de la salle d'exposition 2.

Ces mesures continues peuvent également permettre de mesurer le MMAD moyen des allergènes présents dans la salle d'exposition 2 pendant toute la durée de l'exposition, ou de connaitre sa valeur à chaque instant selon le dispositif de comptage utilisé.

Lorsqu'une mesure est réalisée au niveau de chaque fauteuil 3, on peut avantageusement connaître les paramètres d'exposition relatifs à chacun de ces endroits et connaître ainsi par exemple la dose totale de particules d'allergènes à laquelle un patient se trouvant dans un fauteuil a été exposé pendant la durée de l'exposition, avec leur distribution granulométrique et leur MMAD..

Si les résultats obtenus lors de la procédure de qualification du procédé 31 ne sont pas conformes aux valeurs de consigne, on procède à des ajustements des différents paramètres de fonctionnement du système et on réitère les mesures de la procédure de qualification jusqu'à l'obtention des valeurs de consigne à la tolérance près.

Selon la nature du résultat non conforme, on peut procéder à un ajustement de la concentration en allergènes C_{composition} de la composition aqueuse d'allergènes 8 et/ou du débit d_{composition} avec lequel on introduit la composition aqueuse d'allergènes 8 dans le dispositif de nébulisation par ultrasons 9.

Lorsque cela est possible avec le matériel utilisé, on peut éventuellement ajuster encore la puissance du dispositif de nébulisation par ultrasons 9.

Lorsque les résultats obtenus sont conformes aux valeurs de consigne et sont reproductibles, la procédure de qualification du procédé 31 est terminée et l'exposition peut commencer.

Grâce au procédé selon l'invention, l'atmosphère chargée en allergènes réalisée dans la salle d'exposition, présente avantageusement une très bonne homogénéité avec une concentration en allergènes et un MMAD des allergènes sensiblement constants et correspondant aux valeurs de consigne choisies à la tolérance près. Un tel résultat ne peut être obtenu avec aucun des systèmes connus de l'art antérieur.

De manière évidente, l'invention ne se limite pas aux modes de réalisation préférentiels décrits précédemment et représentés sur les différentes figures, l'homme du métier pouvant y apporter de nombreuses modifications et imaginer d'autres variantes sans sortir ni de la portée, ni du cadre de l'invention définis par les revendications.

## Revendications

1. Procédé de réalisation, dans une salle d'exposition (2) aux allergènes, d'une atmosphère chargée en allergènes, homogène et dont la concentration en allergènes Cₑₓₚₒₛᵢₜᵢₒₙ et le MMAD (diamètre aérodynamique médian en masse) des allergènes Dₑₓₚₒₛᵢₜᵢₒₙ sont préalablement choisis, procédé **caractérisé**
**en ce qu**'il est mis en oeuvre au moyen d'un dispositif de nébulisation par ultrasons (9), à partir d'une composition aqueuse d'allergènes (8) et d'un flux d'air dépourvu d'allergènes (13) présentant un débit dₐᵢᵣ constant ;
**en ce qu**'il comprend les étapes suivantes :
- on choisit les valeurs Cₑₓₚₒₛᵢₜᵢₒₙ et Dₑₓₚₒₛᵢₜᵢₒₙ que l'on souhaite obtenir à une tolérance près dans l'atmosphère de la salle d'exposition (2),
- en fonction de la valeur Dₑₓₚₒₛᵢₜᵢₒₙ choisie, on détermine la fréquence F à laquelle on doit faire fonctionner le dispositif de nébulisation par ultrasons (9) et la concentration en allergènes C_{composition} que doit présenter la composition aqueuse d'allergènes (8), pour obtenir dans l'atmosphère de la salle d'exposition un MMAD des allergènes correspondant à la valeur Dₑₓₚₒₛᵢₜᵢₒₙ choisie à la tolérance près,
- en fonction de la valeur Cₑₓₚₒₛᵢₜᵢₒₙ choisie, du débit dₐᵢᵣ que présente le flux d'air dépourvu d'allergènes (13), du rendement du dispositif de nébulisation par ultrasons (9) et de la concentration en allergènes C_{composition} déterminée, on détermine le débit d_{composition} avec lequel on doit introduire la composition aqueuse d'allergènes (8) dans le dispositif de nébulisation par ultrasons (9), pour obtenir dans l'atmosphère de la salle d'exposition une concentration en allergènes correspondant à la valeur Cₑₓₚₒₛᵢₜᵢₒₙ choisie à la tolérance près,
- on fournit ou on prépare la composition aqueuse d'allergènes (8) avec la concentration en allergènes C_{composition} déterminée,
- on introduit avec le débit d_{composition} déterminé la composition aqueuse d'allergènes (8) dans le dispositif de nébulisation par ultrasons (9) fonctionnant à la fréquence F déterminée,
- on produit, à partir de la composition aqueuse d'allergènes (8) et au moyen du dispositif de nébulisation par ultrasons (9), un nébulisat humide, formé de gouttelettes de composition aqueuse d'allergènes en suspension dans l'air,
- on sèche au moyen d'un flux d'air de séchage (30) le nébulisat humide afin d'obtenir un nébulisat sec (10), formé de particules d'allergènes solides en suspension dans l'air,
- on mélange, en dehors de la salle d'exposition (2), le nébulisat sec (10) au flux d'air dépourvu d'allergènes (13) présentant le débit dₐᵢᵣ, pour obtenir un flux d'air chargé en allergènes (20),
- on introduit dans la salle d'exposition (2) le flux d'air chargé en allergènes (20), à travers au moins une bouche de diffusion (21) située en partie haute de la salle d'exposition (2),
- on aspire le flux d'air chargé en allergènes (20) hors de la salle d'exposition (2) à travers au moins une bouche d'évacuation (22) située en partie basse de la salle d'exposition.

2. Procédé selon la revendication 1 **caractérisé en ce qu'**on réalise des mesures particulaires en continu afin de surveiller le non-dépassement de seuils de sécurité.

3. Procédé selon la revendication 1 **caractérisé en ce que** l'on réalise une procédure de qualification (31) qui comprend au moins une des étapes suivantes :
- on mesure la concentration et le MMAD des allergènes dans l'atmosphère de la salle d'exposition (2) afin de contrôler qu'ils correspondent respectivement aux valeurs Cₑₓₚₒₛᵢₜᵢₒₙ et Dₑₓₚₒₛᵢₜᵢₒₙ choisies à la tolérance près ;
- on mesure le rendement du dispositif de nébulisation par ultrasons (9) dans les conditions de fonctionnement correspondant à la mise en oeuvre du procédé ;
- on utilise plusieurs bouches d'évacuation (22) réparties à différents endroits en partie basse de la salle d'exposition et on choisit le nombre, l'emplacement et/ou l'ouverture des bouches d'évacuation (22) utilisées pour aspirer le flux d'air chargé en allergènes (20), afin d'obtenir une diffusion homogène dudit flux d'air chargé en allergènes (20) dans l'ensemble de la salle d'exposition (2).

4. Procédé selon l'une quelconque des revendications précédentes **caractérisé en ce que** l'on prépare la composition aqueuse d'allergènes (8) avec la concentration en allergènes C_{composition} déterminée, et **en ce que** l'on vérifie sa concentration en allergènes par la technique ELISA.

5. Procédé selon la revendication 3 **caractérisé en ce que** la mesure du rendement du dispositif de nébulisation par ultrasons (9) se fait par prélèvement du nébulisat sec (10) pendant une durée déterminée ; par mise en solution du nébulisat sec (10) prélevé puis mesure de la concentration C_{prélèvement} de cette solution par la technique ELISA ; par calcul de la quantité d'allergènes se trouvant dans le prélèvement à partir de la concentration C_{prélèvement} mesurée ; et par comparaison de la quantité d'allergènes se trouvant dans le prélèvement avec la quantité d'allergènes introduite dans le dispositif de nébulisation par ultrasons (9) pendant la même durée, calculée à partir de la concentration C_{composition} et du débit d_{composition}.

6. Procédé selon la revendication 3 **caractérisé en ce que** la mesure de la concentration des allergènes dans l'atmosphère de la salle d'exposition (2) est réalisée par prélèvement aéraulique puis par dosage du prélèvement par la technique ELISA.

7. Procédé selon la revendication 3 **caractérisé en ce qu'**on procède à des ajustements de la concentration en allergènes C_{composition} ou du débit d_{composition}, si la concentration ou le MMAD des allergènes mesurés dans l'atmosphère de la salle d'exposition (2) pendant la procédure de qualification ne correspondent pas respectivement aux valeurs Cₑₓₚₒₛᵢₜᵢₒₙ ou Dₑₓₚₒₛᵢₜᵢₒₙ choisies à la tolérance près.

8. Procédé selon l'une quelconque des revendications précédentes **caractérisé en ce que** l'on mesure au niveau de chaque fauteuil (3) équipant la salle d'exposition (2), la concentration des allergènes dans l'atmosphère de la salle d'exposition ou la dose totale de particules d'allergènes reçues à cet endroit pendant la durée de l'exposition.

9. Système d'exposition aux allergènes (1) comprenant une salle d'exposition (2) aux allergènes, et apte à produire dans cette salle d'exposition (2) une atmosphère chargée en allergènes, homogène et dont la concentration en allergènes Cₑₓₚₒₛᵢₜᵢₒₙ et le MMAD (diamètre aérodynamique médian en masse) des allergènes Dₑₓₚₒₛᵢₜᵢₒₙ peuvent être préalablement choisis, système d'exposition aux allergènes (1) **caractérisé en ce qu'**il comprend :
- un dispositif de nébulisation par ultrasons (9), réglé pour travailler à une fréquence F adaptée par rapport à la valeur de Dₑₓₚₒₛᵢₜᵢₒₙ choisie, et comprenant une zone de préparation (34) d'un nébulisat humide à partir d'une composition aqueuse d'allergènes (8), et une entrée d'air de séchage (35) par laquelle un flux d'air de séchage (30) est amené au contact du nébulisat humide pour le sécher et former ainsi un nébulisat sec (10) d'allergènes ;
- une composition aqueuse d'allergènes (8) préparée ou fournie avec une concentration C_{composition} dépendant de la valeur Dₑₓₚₒₛᵢₜᵢₒₙ choisie et de la fréquence F ;
- un moyen d'introduction, avec un débit réglable, de la composition aqueuse d'allergènes (8) dans le dispositif de nébulisation par ultrasons (9) ;
- une zone de mélange (14), séparée de la salle d'exposition (2), communiquant d'une part avec la sortie du dispositif de nébulisation par ultrasons (9) et d'autre part avec une arrivée d'un flux d'air dépourvu d'allergènes (13) de débit constant, et dans laquelle est réalisé le mélange entre le nébulisat sec (10) et le flux d'air dépourvu d'allergènes (13) pour obtenir un flux d'air chargé en allergènes (20);
- au moins une bouche de diffusion (21) située en partie haute de la salle d'exposition (2), qui communique avec la chambre de mélange (14) et par laquelle le flux d'air chargé en allergènes (20) pénètre dans la salle d'exposition (2) ;
- plusieurs bouches d'évacuation (22), permettant d'aspirer le flux d'air chargé en allergènes (20) hors de la salle d'exposition (2), réparties à différents endroits en partie basse de la salle d'exposition (2), et dont l'ouverture peut être fermée ou réglée indépendamment les unes des autres.

10. Système d'exposition aux allergènes (1) selon la revendication 9 **caractérisé en ce qu'**il comporte au moins un dispositif de mesure particulaire (24) permettant de surveiller en continu le non-dépassement de seuils de sécurité ou de mesurer le MMAD des allergènes dans l'atmosphère de la salle d'exposition (2).

11. Système d'exposition aux allergènes (1) selon la revendication 10 **caractérisé en ce que** le dispositif de mesure particulaire (24) est un compteur de particules (33) par laser ou par caméra.

12. Système d'exposition aux allergènes (1) selon l'une quelconque des revendications 9 à 11 **caractérisé en ce qu'**il comporte un collecteur d'aérosol permettant de réaliser un prélèvement du nébulisat sec (10) par prélèvement aéraulique, ou une plaque froide permettant de réaliser un prélèvement du nébulisat sec (10) par condensation de celui-ci.

13. Système d'exposition aux allergènes (1) selon l'une quelconque des revendications 9 à 12 **caractérisé en ce qu'**il comporte au moins un dispositif (36) de mesure de la concentration des allergènes dans l'atmosphère de la salle d'exposition (2).

## Patentansprüche

1. Verfahren zum Erzeugen einer homogenen, allergenversetzten Atmosphäre in einem Allergen-Expositionsraum (2), bei dem die Allergenkonzentration Cₑₓₚₒₛᵢₜᵢₒₙ und der MMAD (massenbezogener medianer aerodynamischer Durchmesser) der Expositionsallergene vorher ausgewählt werden, das Verfahren ist **dadurch gekennzeichnet,**
**dass** es umgesetzt wird mit Hilfe eines Ultraschallverneblers (9), unter Verwendung einer wässrigen Allergenzusammensetzung (8) und einem allergenfreien Luftstrom (13) mit einem konstanten Luftdurchsatz;
**dass** das Verfahren die folgenden Schritte umfasst:
- Auswahl der Werte Cₑₓₚₒₛᵢₜᵢₒₙ und Dₑₓₚₒₛᵢₜᵢₒₙ, die bis auf eine Toleranz in der Atmosphäre des Expositionsraums (2) erreicht werden sollen,
- in Abhängigkeit vom gewählten Wert Dₑₓₚₒₛᵢₜᵢₒₙ wird die Frequenz F gewählt, bei der der Ultraschallvernebler (9) arbeiten werden soll, sowie die Allergenkonzentration C_{composition}, die die wässrige Allergenzusammensetzung (8) aufweisen muss, um in der Atmosphäre des Expositionsraumes einen Allergen- MMAD mit einem Wert entsprechend bis auf die Toleranz dem ausgewählte Wert Dₑₓₚₒₛᵢₜᵢₒₙ zu erhalten
- in Abhängigkeit vom gewählten Wert Cₑₓₚₒₛᵢₜᵢₒₙ, dem Luftdurchsatz des allergenfreien Luftstroms (13), der Leistung des Ultraschallverneblers (9) und der festgelegten Allergenkonzentration C_{composition} wird die Menge D_{composition} bestimmt, mit dem die wässrige Allergenzusammensetzung (8) in den Ultraschallvernebler (9) eingeführt werden muss, um im Expositionsraum eine Allergenkonzentration zu erreichen, die dem ausgewählten Wert C_{composition} bis auf die Toleranz entspricht,
- die wässrige Allergenzusammensetzung (8) wird mit der festgelegten Allergenkonzentration C_{composition} geliefert oder hergestellt,
- die wässrige Allergenzusammensetzung (8) D_{composition} wird in der festgelegten Menge in den Ultraschallvernebler (9) eingeführt, der mit der festgelegten Frequenz F arbeitet,
- ausgehend von der wässrigen Allergenzusammensetzung (8) wird mittels des Ultraschallverneblers (9) ein feuchter Sprühnebel erzeugt, bestehend aus Tröpfchen der wässrigen Allergenzusammensetzung, die sich in Suspension in der Luft befinden,
- mittels eines Trocknungsluftstroms (30) wird der feuchte Sprühnebel getrocknet, um einen trockenen Sprühnebel (10) zu erhalten, der aus festen Allergenpartikeln besteht, die sich in Suspension in der Luft befinden,
- Außerhalb des Expositionsraumes (2) findet eine Vermischung des trockenen Sprühnebels (10) mit dem allergenfreien Luftstrom (13) und dem entsprechenden Luftdurchsatz, um den mit Allergenen versetzten Luftstrom (20) zu erhalten,
- in den Expositionsraum (2) wird der mit Allergenen versetzte Luftstrom (20) über mindestens eine Diffusionsdüse (21) im oberen Teil des Expositionsraumes (2) eingeblasen
- der mit Allergenen versetzte Luftstrom (20) wird aus dem Expositionsraum (2) über mehrere bodennahe Auslässe (22) abgesaugt.

2. Verfahren gemäß Anspruch 1, **dadurch gekennzeichnet, dass** kontinuierlich Partikelmessungen durchgeführt werden, um zu überwachen, dass die Sicherheitsschwellen nicht überschritten werden.

3. Verfahren gemäß Anspruch 1, **dadurch gekennzeichnet, dass** ein Qualifikationsverfahren (31) durchgeführt wird, das mindestens einen der folgenden Schritte enthält:
- Messung der Konzentration und des MMAD der Allergene in der Atmosphäre des Expositionsraums (2) um zu kontrollieren, dass sie jeweils den ausgewählten Werten Cₑₓₚₒₛᵢₜᵢₒₙ und Dₑₓₚₒₛᵢₜᵢₒₙ bis auf die Toleranz entsprechen;
- Messung der Leistung des Ultraschallverneblers (9) unter den Betriebsbedingungen, die dem Einsatz des Verfahrens entsprechen;
- Verwendung verschiedener Absaugdüsen (22), verteilt an verschiedenen Stellen im unteren Bereich des Expositionsraums und Auswahl der Anzahl, Position und/oder Öffnung der Absaugdüsen (22) zum Ansaugen des mit Allergenen versetzten Luftstroms (20) um eine homogene Verteilung dieses mit Allergenen versetzte Luftstroms (20) im ganzen Expositionsraum (2) zu erreichen.

4. Verfahren gemäß irgendeinem der vorhergehenden Ansprüche, **dadurch gekennzeichnet, dass** die wässrige Allergenzusammensetzung (8) mit der festgelegten Allergenkonzentration C_{composition} vorbereitet wird und dass die Allergenkonzentration durch die ELISA-Technik überprüft wird.

5. Verfahren nach Anspruch 3, **dadurch gekennzeichnet, dass** die Messung der Leistung des Ultraschallverneblers (9) durch Probennahme vom trockenen Sprühnebel (10) während einer bestimmten Dauer erfolgt; durch Lösung der Probe des trockenen Sprühnebels (10), dann Messung der Konzentration C_{prélèvement} durch die ELISA-Technik; durch Berechnung der Allergenmenge, die sich in der Probe befinden, ausgehend von der gemessenen Konzentration C_{prélèvement}; und durch Vergleich der in der Probe befindlichen Allergenmenge mit der in den Ultraschallvernebler (9) während derselben Zeit eingeführten Allergenmenge, ausgehend von der Konzentration C_{composition} und dem Durchsatz D_{composition}.

6. Verfahren gemäß Anspruch 3, **dadurch gekennzeichnet, dass** die Messung der Allergenkonzentration in der Atmosphäre des Expositionsraums (2) durch lufttechnische Probenahme erfolgt, dann durch Dosierung der Probe mittels ELISA-Technik.

7. Verfahren gemäß Anspruch 3, **dadurch gekennzeichnet, dass** Anpassungen der Allergenkonzentration C_{composition} oder des Durchsatzes D_{composition} vorgenommen werden, sobald die während des Beurteilungsverfahrens in der Atmosphäre des Expositionsraums (2) gemessene Konzentration oder der MMAD der Allergene nicht jeweils den gewählten Werten Cₑₓₚₒₛᵢₜᵢₒₙ oder Dₑₓₚₒₛᵢₜᵢₒₙ bis auf die Toleranz entsprechen.

8. Verfahren nach irgendeinem der vorstehenden Ansprüche, **dadurch gekennzeichnet, dass** in Höhe jedes Sessels (3), mit denen der Expositionsraum (2) ausgestattet ist, die Allergenkonzentration in der Atmosphäre des Expositionsraums oder die Gesamtdosis der an dieser Stelle während der Dauer der Exposition ankommenden Allergenpartikel gemessen wird.

9. System der Allergenexposition (1), umfassend einen Allergen-Expositionsraum (2), das in der Lage ist, in diesem Expositionsraum (2) eine allergengeladene, homogene Atmosphäre zu erzeugen und bei dem die Allergenkonzentration C_{composition} und der MMAD (massenbezogener medianer aerodynamischer Durchmesser) der Expositionsallergene Dₑₓₚₒₛᵢₜᵢₒₙ vorher ausgewählt werden können; System der Allergenexposition (1), **dadurch gekennzeichnet, dass** es umfasst:
- einen Ultraschallvernebler (9), so eingestellt, dass er mit einer Frequenz F arbeitet, die bezogen auf den gewählten Wert Dₑₓₚₒₛᵢₜᵢₒₙ angepasst wird und dass es eine Vorbereitungszone (34) eines feuchten Sprühnebels umfasst, ausgehend von einer wässrigen Allergenzusammensetzung (8) und einen Einlass für trockene Luft (35) durch die ein Trocknungsluftstrom (30) in Kontakt mit dem feuchten Sprühnebel gebracht wird um ihn zu trocknen und so einen trockenen Allergen- Sprühnebel (10) zu erzeugen;
- eine wässrige Allergenzusammensetzung (8) geliefert oder hergestellt, mit einer Konzentration C_{composition}, die vom Wert Dₑₓₚₒₛᵢₜᵢₒₙ und der Frequenz F abhängt;
- eine Zuführvorrichtung mit einstellbarem Durchsatz der wässrigen Allergenzusammensetzung (8) im Ultraschallvernebler (9);
- eine Mischzone (14), getrennt vom Expositionsraum (2), die in Verbindung steht mit erstens dem Auslass des Ultraschallverneblers (9) und zweitens einem Einlass für einen allergenfreien Luftstrom (13) mit konstantem Durchsatz und in dem eine Mischung zwischen dem trockenen Sprühnebel (10) und dem allergenfreien Luftstrom (13) hergestellt wird, um einen allergengeladenen Luftstrom (20) zu erzeugen;
- mindestens eine Diffusionsdüse (21) im oberen Teil des Expositionsraumes (2), die mit der Mischung (14) in Verbindung steht und durch die der allergengeladenen Luftstrom (20) in den Expositionsraum (2) einströmt;
- mehrere Absaugdüsen (22), mit denen der allergengeladene Luftstrom (20) aus dem Expositionsraum (2) abgesaugt werden, kann, verteilt an verschiedenen Stellen im Expositionsraum (2), deren Öffnung unabhängig voneinander geschlossen oder gesteuert werden kann.

10. Allergenexpositionssystem (1) gemäß Anspruch 9 **dadurch gekennzeichnet, dass** es mindestens eine Vorrichtung zur Partikelmessung (24) enthält, mit der ständig die Nichtüberschreitung der Sicherheitsschwellen überwacht oder die Allergen-MMAD in der Atmosphäre des Expositionsraumes (2) gemessen werden kann.

11. Allergenexpositionssystem (1) gemäß Anspruch 10 **dadurch gekennzeichnet, dass** es sich bei der Vorrichtung zur Partikelmessung (24) um einen Laser- oder Kamera-Partikelzähler (33) handelt.

12. Allergenexpositionssystem (1) gemäß einem der Ansprüche 9 bis 11, **dadurch gekennzeichnet, dass** es einen Aerosol-Sammler enthält, mit dem Proben des trockenen Sprühnebels (10) durch lufttechnische Probennahme entnommen werden können, oder eine Kühlplatte, mit der eine Probe des trockenen Sprühnebels (10) durch Kondensation genommen werden kann.

13. Allergenexpositionssystem (1) gemäß einem der Ansprüche 9 bis 12, **dadurch gekennzeichnet, dass** er mindestens eine Messvorrichtung (36) der Allergenkonzentration in der Atmosphäre des Expositionsraumes (2) enthält.

## Claims

1. Method of creating an homogeneous allergen-laden atmosphere in an exposure room (2) whose concentration in allergens Cₑₓₚₒₛᵤᵣₑ and MMAD (mass median aerodynamic diameter) of the allergens Dₑₓₚₒₛᵤᵣₑ are previously chosen, which method is **characterized**
**in that** implementation is carried out by means of an ultrasound nebulization device (9), from an aqueous composition of allergens (8) and an airflow devoid of allergens (13) having a constant flowrate dₐᵢᵣ;
**in that**, it includes the following steps:
- the values Cₑₓₚₒₛᵤᵣₑ and Dₑₓₚₒₛᵤᵣₑ, to be obtained to within a tolerance in the atmosphere of the exposure room (2), are chosen;
- depending on the chosen value Dₑₓₚₒₛᵤᵣₑ, the frequency F at which the ultrasound nebulization device (9) is to be made to operate and the concentration in allergens C_{composition} that the aqueous composition of allergens (8) must have, to obtain in the atmosphere of the exposure room an allergen MMAD corresponding to the chosen value Dₑₓₚₒₛᵤᵣₑ to within the tolerance, are determined,
- depending on the chosen value Cₑₓₚₒₛᵤᵣₑ, on the flow rate dₐᵢᵣ of the airflow devoid of allergens (13), on the efficiency of the ultrasound nebulization device (9) and on the determined allergen composition C_{composition}, the flowrate d_{composition}, with which the aqueous composition of allergens (8) is to be introduced into the ultrasound nebulization device (9) to obtain in the atmosphere of the exposure room an allergen concentration corresponding to the chosen value Cₑₓₚₒₛᵤᵣₑ to within the tolerance, is determined,
- the aqueous composition of allergens (8) is supplied or prepared with the determined allergen concentration C_{composition},
- the aqueous composition of allergens (8) is introduced, with the determined flowrate d_{composition}, into the ultrasound nebulization device (9) operating at the detemined frequency F,
- from the aqueous composition of allergens (8) and by means of the ultrasound nebulization device (9), a moist nebulisate is formed, made up of droplets of the aqueous composition of allergens in suspension in the air,
- the moist nebulisate is dried by means of a drying airflow (30) to obtain a dry nebulisate (10), made up of solid allergen particles in suspension in the air,
- the dry nebulisate (10) is mixed, outside the exposure room (2), with the airflow devoid of allergens (13) having the flowrate dₐᵢᵣ, to obtain an allergen-laden airflow (20),
- the allergen-laden airflow (20) is introduced into the exposure room (2), through at least one diffusion vent (21) located in the upper part of the exposure room (2),
- the allergen-laden airflow (20) is drawn out of the exposure room (2) through at least one exhaust vent (22) located in the lower part of the exposure room.

2. Method according to claim 1 **characterized in that** continuous particulate measurements are carried out to monitor that safety thresholds are not exceeded.

3. Method according to claim 1 **characterized in that** a qualification procedure (31) is carried out which comprises at least one of the following steps:
- the concentration and MMAD of the allergens in the atmosphere of the exposure room (2) are measured to check that they correspond respectively to the chosen values Cₑₓₚₒₛᵤᵣₑ and Dₑₓₚₒₛᵤᵣₑ to within the tolerance;
- the efficiency of the ultrasound nebulization device (9) is measured under the operating conditions corresponding to the implementation of the method;
- several exhaust vents (22) distributed at different locations in the lower part of the exposure room are used and the number, location and/or opening of the exhaust vents (22) used to draw out the allergen-laden airflow (20) is chosen, to obtain an homogeneous diffusion of said allergen-laden airflow (20) throughout the exposure room (2).

4. Method according to any one of the previous claims **characterized in that** the aqueous composition of allergens (8) is prepared with the determined allergen concentration C_{composition} and **in that** its allergen concentration is verified using the ELISA technique.

5. Method according to claim 3, **characterized in that** the efficiency of the ultrasound nebulization device (9) is measured by collecting the dry nebulisate (10) for a determined period of time; by dissolving the collected dry nebulisate (10) and then measuring the concentration Cₛₐₘₚₗₑ of this solution by the ELISA technique; by calculating the quantity of allergens present in the sample from the measured concentration Cₛₐₘₚₗₑ; and by comparing the quantity of allergens present in the sample with the quantity of allergens introduced into the ultrasound nebulization device (9) during the same period, calculated from the concentration C_{composition} and the flowrate d_{composition}.

6. Method according to claim 3 **characterized in that** the measurement of the concentration of allergens in the atmosphere of the exposure room (2) is carried out by air sampling and then by assaying the sample using the ELISA technique.

7. Method according to claim 3, **characterized in that** adjustment is made of the concentration of allergens C_{composition} or of the flow rate d_{composition}, if the concentration or MMAD of the allergens measured in the atmosphere of the exposure room (2) during the qualification procedure do not correspond respectively to the chosen values Cₑₓₚₒₛᵤᵣₑ or Dₑₓₚₒₛᵤᵣₑ to within the tolerance.

8. Method according to any one of the above claims, **characterized in that** the concentration of allergens in the atmosphere of the exposure room or the total dose of allergen particles received at this location during the exposure period is measured at the level of each chair (3) fitted in the exposure room (2).

9. Allergen exposure system (1) comprising an allergen exposure room (2) and able to produce in this exposure room (2) an alergen-laden atmosphere which is homogeneous and whose concentration in allergens Cₑₓₚₒₛᵤᵣₑ and MMAD (mass median aerodynamic diameter) of the allergens Dₑₓₚₒₛᵤᵣₑ can be chosen previously, said allergen exposure system (1) is **characterized in that** it comprises:
- An ultrasound nebulization device (9) set to operate at a frequency F adapted to the chosen value Dₑₓₚₒₛᵤᵣₑ and comprising a preparation area (34) of a moist nebulisate from an aqueous composition of allergens (8), and a drying air inlet (35) through which a drying airflow (30) is brought into contact with the moist nebulisate to dry it and thereby form a dry nebulisate (10) of allergens.
- an aqueous composition of allergens (8) prepared or supplied with a concentration C_{composition} depending on the chosen value Dₑₓₚₒₛᵤᵣₑ and the frequency F.
- a means for introducing, with an adjustable flowrate, the aqueous composition of allergens (8) in the ultrasound nebulization device (9);
- a mixing zone (14), separated from the exposure room (2), connecting on the one hand with the outlet of the ultrasound nebulization device (9) and on the other hand with an inlet of an airflow devoid of allergens (13) with a constant flowrate, and in which the dry nebulisate (10) and the airflow devoid of allergens (13) are mixed to obtain an allergen-laden airflow (20),
- at least one diffusion vent (21) located in the upper part of the exposure room (2), which connects with the mixing chamber (14) and through which the allergen-laden airflow (20) enters the exposure room (2);
- several exhaust vents (22), allowing the allergen-laden airflow (20) to be drawn out of the exposure room (2), distributed at different places in the lower part of the exposure room (2), and whose opening can be closed or adjusted independently of each other.

10. Allergen exposure system (1) according to claim 9 **characterized in that** it comprises at least one particulate measuring device (24) to continuosly monitor that safety thresholds are not exceeded or to mesure the MMAD of the allergens in the atmosphere of the exposure room (2).

11. Allergen exposure system (1) according to claim 10 **characterized in that** the particulate measuring device (24) is a laser or camera particle counter (33).

12. Allergen exposure system (1) according to any one of claims 9 to 11, **characterized in that** it includes an aerosol collector for sampling the dry nebulisate (10) by air sampling, or a cold plate for sampling the dry nebulisate (10) by its condensation.

13. Allergen exposure system (1) according to any one of claims 9 to 12, **characterized in that** it includes at least one device (36) for measuring the concentration of allergens in the atmosphere of the exposure room (2)
